# EUROPEAN PATENT SPECIFICATION

(11) **EP 3 639 037 B1**
(45) Date of publication and mention of the grant of the patent: **13.10.2021**
(21) Application number: 18731089.1
(22) Date of filing: 13.06.2018
(51) Int. Cl.: G01N 33/68

(54) **METHOD FOR DIAGNOSING STROKE USING A COMBINATION OF BIOMARKERS**
VERFAHREN ZUR DIAGNOSE EINES SCHLAGANFALLS UNTER VERWENDUNG EINER KOMBINATION VON BIOMARKERN
PROCÉDÉ DE DIAGNOSTIC D'ACCIDENT VASCULAIRE CÉRÉBRAL UTILISANT UNE COMBINAISON DE BIOMARQUEURS

(30) Priority: 14.06.2017 GB 201709475
(43) Date of publication of application: 22.04.2020
(73) Proprietor: Randox Laboratories Ltd, Crumlin, Antrim BT29 4QY (GB); Randox Teoranta, Letterkenny, Co. Donegal F94 TV06 (IE)
(72) Inventor: MCCONNELL, Ivan, Crumlin Antrim BT29 4QY (GB); FITZGERALD, Peter, Crumlin Antrim BT29 4QY (GB); LAMONT, John, Crumlin Antrim BT29 4QY (GB); RICHARDSON, Ciaran, County Donegal Dungloe F94 TV06 (IE)
(86) International application number: PCT/EP2018/065688
(87) International publication number: WO 2018/229144

(56) References cited:
- US-A1- 2015 126 385
- US-A1- 2016 139 147
- ALLARD LAURE ET AL: "PARK7 and nucleoside diphosphate kinase A as plasma markers for the early diagnosis of stroke", CLINICAL CHEMISTRY, AMERICAN ASSOCIATION FOR CLINICAL CHEMISTRY, WASHINGTON, DC, vol. 51, no. 11, 1 November 2005 (2005-11-01), pages 2043-2051, XP002547809, ISSN: 0009-9147, DOI: 10.1373/CLINCHEM.2005.053942
- WALTER AGENO ET AL: "Plasma Measurement of D-Dimer Levels for the Early Diagnosis of Ischemic Stroke Subtypes", ARCHIVES OF INTERNAL MEDICINE., vol. 162, no. 22, 9 December 2002 (2002-12-09), page 2589, XP055492750, US ISSN: 0003-9926, DOI: 10.1001/archinte.162.22.2589

## Description

### Background to the Invention

Stroke is the third leading cause of death worldwide and can be defined as the rapidly developing loss of brain function(s) due to interruption in the blood supply to the brain. According to the World Health Organisation, 15 million people per year suffer stroke worldwide, with 5 million dying and a further 5 million being permanently disabled. High blood pressure is estimated to be a contributing factor in 12.7 million of these 15 million stroke cases. In the UK, approximately 150,000 people have a stroke each year and stroke accounts for around 53,000 deaths per year. Stroke costs the economy an estimated £8 billion per year in England alone and stroke patients occupy approximately 20% of all acute hospital beds and 25% of long term beds.

Stroke can be classified into three sub-types:
i) Ischaemic stroke (IS) occurs when blood supply to the brain is decreased, resulting in brain damage. An ischemic stroke occurs when a blood vessel becomes blocked, usually via a blood clot. This clot may form locally at an atherosclerotic plaque (thrombotic stroke) or alternatively may occur due to a travelling particle or debris that has originated from elsewhere in the bloodstream (embolic stroke);
ii) Transient ischaemic attack (TIA) is a 'mini stroke' that occurs when blood supply to the brain is temporarily decreased. A TIA is diagnosed if symptoms are quickly resolved (within 24 hours with the individual returning to normal health); and
iii) Haemorrhagic stroke (HS) occurs when blood accumulates within the skull vault, usually when a weakened blood vessel ruptures. Haemorrhagic stroke can be classified into two major subtypes, namely intracerebral (within the brain tissue) and subarachnoid (around the surface of the brain and under its protective layer).

IS and TIA account for approximately 85% of all stroke cases and HS accounts for 15%. In order to minimise neurological damage following stroke it is crucial that stroke patients are rapidly and accurately diagnosed, so that appropriate treatment can be administered. For example, in order to break down clots thrombolytic therapy such as tissue plasminogen activator (TPA) can be administered. However, such therapy is only warranted in IS and is detrimental in HS. The nature of TIA does not require such therapy and blood thinners such as warfarin and aspirin are prescribed in such cases.

At present, if stroke is suspected, physical symptoms are evaluated and a computerised tomography (CT) scan is usually performed. A CT scan has good sensitivity for identifying HS patients (approximately 90% sensitivity) but poor sensitivity for identifying IS and TIA patients (approximately 20% sensitivity). In practice minimal or no tissue damage occurs for TIA due to its transient nature, therefore CT scanning is ineffective as a diagnostic technique. Magnetic Resonance Imaging (MRI) has improved sensitivity for IS diagnosis (up to approximately 80%) but increased time requirements, machine accessibility, and high cost have limited its use for stroke diagnosis. The poor sensitivity of CT scanning for the detection of IS and TIA means that a biological fluid-based diagnostic biomarker tests for detecting IS and TIA would be an invaluable tool to aid clinicians in the diagnosis of stroke sub-type. Biological fluid-based biomarkers have the potential to expedite and increase the accuracy of stroke diagnosis. Various candidate biomarkers have been proposed for the diagnosis of stroke and stroke sub-type delineation and there are several descriptions of IS/TIA versus HS discrimination in the prior art, for example EP1238284, WO2010/086697, WO 2010/012834, and WO 2002/012892. EP1419388 discloses data that distinguishes IS from HS and all stroke types from non-stroke controls. US2015126385 discloses data for aiding the diagnosis of ischaemic stroke based on determining the concentration of vascular cell adhesion molecule-1 (VCAM-1) and one or more additional biomarkers selected from heart-type fatty acid binding protein (h-FABP), interleukin 6 (IL-6) and C-reactive protein (CRP). However, none have thus far found use in clinical practice and there is a real clinical need for biomarkers of all three stroke sub-types that have high sensitivity and specificity to enable accurate diagnosis. Differential diagnosis between the three different stroke sub-types using a blood test would facilitate a more informed clinical decision, potentially render unnecessary expensive and less expeditious neuroimaging diagnostics, and could improve the clinical outcome for patients.

### Summary of the Invention

1. A method of aiding the diagnosis of stroke in a patient suspected of having had or currently suffering a stroke, said method comprising;
   i) Determining the concentration of Parkinson disease protein 7 (PARK7) and D-dimer in an ex vivo blood, serum or plasma sample obtained from the patient;
   ii) Establishing the statistical significance of the concentration of the biomarkers by comparison to a control value, wherein each of the biomarker concentration values are inputted into a statistical algorithm or algorithms to produce an output value that correlates with diagnosis of stroke.
2. The method of claim 1 wherein the concentration of one or more additional biomarker selected from the list consisting of Fatty acid-binding protein 3 (FABP3), Glial fibrillary acid protein (GFAP), Glutathione S-transferase P (GSTP), interleukin 6 (IL-6), Nucleoside diphosphate kinase A (NDKA) and soluble tumour necrosis factor receptor 1 (sTNFr1) is also determined.
3. The method according to claim 2, wherein any of the following concentrations are determined
   i) PARK7, D-dimer and FABP3
   ii) PARK7, D-dimer and IL-6
   iii) PARK7, D-dimer and sTNFr1
   iv) PARK7, D-dimer and GFAP
   v) PARK7, D-dimer, FABP3 and sTNFr1
   vi) PARK7, D-dimer, FABP3 and IL-6
   vii) PARK7, D-dimer, FABP3, sTNFr1 and IL-6
   viii) D-dimer, FABP3, GFAP and PARK7
   ix) D-dimer, FABP3, GFAP, IL-6, PARK7 and sTNFr1
4. The method according to claims 1-3 for the diagnosis of ischaemic stroke.

### Detailed Description

Unless otherwise stated technical terms are used according to the conventional usage as known to those skilled in the art.

The present invention relates to biomarker-based methods and biochips that can be used to aid in the diagnosis of stroke and the discrimination between haemorrhagic stroke (HS), ischemic stroke (IS), transient ischemic attack (TIA) and stroke mimics.

Unless stated otherwise, all references herein to the term 'stroke' encompasses all three forms of stroke; haemorrhagic stroke (HS), ischaemic stroke (IS) and transient ischaemic attack (TIA). As used herein, the term 'ischaemic stroke (IS)' refers to the type of stroke that occurs when blood supply to the brain is decreased, resulting in brain damage. An ischemic stroke occurs when a blood vessel becomes blocked, usually via a blood clot. This clot may form locally at an atherosclerotic plaque (thrombotic stroke) or alternatively may occur due to a travelling particle or debris that has originated from elsewhere in the bloodstream (embolic stroke). The term 'transient ischaemic attack (TIA)' refers to a 'mini stroke' that occurs when blood supply to the brain is temporarily decreased. A TIA is diagnosed if symptoms are quickly resolved (within 24 hours with the individual returning to normal health). The term 'haemorrhagic stroke (HS)' occurs when blood accumulates within the skull vault, usually when a weakened blood vessel ruptures. Haemorrhagic stroke can be classified into two major sub-types: intracerebral (within the brain tissue); and subarachnoid (around the surface of the brain and under its protective layer). The term 'stroke mimics' as used herein refers to any nonvascular disease that presents with stroke-like symptoms, including but not limited to hypoglycaemia, hyperglycemia, epilepsy, multiple sclerosis, hemiplegic migraine, and intracranial tumours or infections, such as meningitis, encephalitis and abscesses.

The present invention provides a method of aiding the diagnosis of stroke in a patient suspected of having had or currently suffering a stroke, comprising: Determining the concentration of PARK7 and D-dimer in an ex vivo sample obtained from the patient; and establishing the significance of the concentration of the biomarkers. In a further embodiment the concentration of one or more additional biomarker selected from the list consisting of FABP3, GFAP, GSTP, IL-6, NDKA and sTNFr1 is also determined. Preferred combinations of markers for the diagnosis of stroke include PARK7, D-dimer and FABP3, PARK7, D-dimer and IL-6, PARK7, D-dimer and sTNFr1, PARK7, D-dimer and GFAP, PARK7, D-dimer, FABP3 and sTNFr1, PARK7, D-dimer, FABP3 and IL-6, PARK7, D-dimer, FABP3, sTNFr1 and IL-6, D-dimer, FABP3, GFAP and PARK7 and D-dimer, FABP3, GFAP, IL-6, PARK7 and sTNFr1.

The term "D-dimer" as used herein refers to the fibrin degradation product. The term "sTNFR1" as used herein refers to soluble Tumour Necrosis factor Receptor 1 (UniProt P19438). The term "IL-6" as used herein refers to interleukin 6 (UniProt P05231). The term "PARK7" as used herein refers to Parkinson disease protein 7, also known as DJ-1 (UniProt Q99497). The term "FABP3" as used herein refers to fatty acid-binding protein 3 (UniProt P05413). The term "GFAP" as used herein refers to Glial fibrillary acid protein (UniProt P14136). The term "GSTP" as used herein refers to Glutathione S-transferase P (UniProt P09211). The term "NDKA" as used herein refers to Nucleoside diphosphate kinase A (Uniprot P15531).

It is possible that levels of some non-brain specific biomarkers are altered in stroke mimics, therefore these markers, when used in isolation, may result in false positive results for the diagnosis of stroke when levels are compared to healthy control/reference groups. In such cases combination with a brain specific marker of which levels are not altered in stroke mimics may improve the clinical accuracy of the diagnostic method. The levels of the brain specific biomarker can be compared to levels in healthy control groups or stroke mimic control groups to aid in the differentiation of such conditions. The reference group can also consist of both healthy controls and stroke mimics grouped together. In the current invention it was found that adding the brain-specific marker PARK7 to D-dimer assays improved the diagnostic performance. Table 1 shows the location and pathophysiological role of the four brain-specific biomarkers, PARK7, GSTP, NDKA and GFAP, and four non-brain-specific biomarkers, D-dimer, FABP3, IL-6 and sTNFr1, of the current invention.

Therefore, the concentrations of one or more biomarkers selected from the list consisting of D-dimer, FABP3, IL-6 and sTNFr1 are compared to levels in a healthy control group. In yet another embodiment the concentrations of one or more biomarkers selected from the list consisting of PARK7, GSTP, NDKA and GFAP are compared to levels in a stroke mimic control group.

**Table 1 The preferred biomarkers of the current invention along with their location within the human body and their pathophysiological role**

| **8-plex Stroke Biochip Array** | **Location - Pathophysiological role** |
|---|---|
| Glutathione S-transferase Pi (GSTP1) | Cytoplasmic neural and glial cell populations-Oxidative stress |
| Parkinson Disease Protein 7 (PARK7) | Cytoplasmic neural and glial cell populations - Oxidative stress |
| Nucleoside Diphosphate Kinase A (NDKA) | Cytoplasmic neural and glial cell populations - Ischemic cascade |
| Glial Fibrillary Acidic Protein (GFAP) | Astrocyte intermediate filament protein - Cytoplasmic stress |
| Soluble Tumour Necrosis Factor Receptor 1 (sTNFr1) | Systemic circulation - Inflammation |
| d-Dimer | Systemic circulation - Fibrinolysis |
| Fatty Acid Binding Protein 3 (FABP3) | Cystolic - Cellular damage |
| Interleukin 6 (IL-6) | Systemic circulation - Inflammation |

References herein to 'a patient suspected of having had or currently suffering a stroke' include a patient who has been diagnosed as currently suffering from a stroke or who has been diagnosed as having previously suffered a stroke and any patient presenting with stroke-like symptoms. The stroke may have been a recent event, such an event having initiated the process of the individual seeking clinical help.

The terms "subject" and "patient" may be used interchangeably herein and refer to a mammal including a non-primate (e.g. a cow, pig, horse, dog, cat, rat and mouse) and a primate (e.g. a monkey and human). Preferably the subject or patient is a human.

As used herein, the term 'biomarker' refers to a molecule present in a biological sample obtained from a patient, the concentration of which in said sample may be indicative of a pathological state. Various biomarkers that have been found by the present inventors to be useful in differentiating between different stroke sub-types, either alone or in combination with other diagnostic methods, or as complementary biomarkers in combination with other biomarkers, are described herein. A used herein, the term 'complementary biomarker' refers to a biomarker that can be used in conjunction with other stroke biomarkers to support diagnosis.

It is well understood in the art that biomarker normal or 'background' concentrations may exhibit slight variation due to, for example, age, gender or ethnic/geographical genotypes. As a result, the cut-off value used in the methods of the invention may also slightly vary due to optimization depending upon the target patient/population.

The biological sample obtained from a patient is preferably a blood, serum or plasma sample. As used herein, the term *'ex vivo'* has its usual meaning in the art and refers to a sample that has been removed from a patient's body. When a blood sample is taken from the patient for analysis, whole blood, serum or plasma is analysed. Analysis of the blood sample can be by way of several analytical methodologies such as mass spectrometry linked to a separation step such as chromatography. The preferred methodology is based on immuno-detection. Immuno-detection technology is also readily incorporated into transportable or hand-held devices for use outside of the clinical environment. A quantitative immunoassay such as a Western blot or ELISA can be used to detect the amount of protein. A preferred method of analysis comprises using a multi-analyte biochip which enables several proteins to be detected and quantified simultaneously. 2D Gel Electrophoresis is also a technique that can be used for multi-analyte analysis.

The skilled person will be aware of numerous suitable methods for developing statistical algorithms. Examples of suitable classification algorithms include multinominal logistic regression, multilayer perceptron neural network (MLP), artificial neural networks, support vector machines, Naïve Bayes and random forest classifiers. The present inventors have found that both multinominal logistic regression and MPL achieve similar performance in the context of the present invention, suggesting the importance of the analytes (i.e. biomarkers) used in the methods of the invention, rather than the method used to generate the algorithmic model. However, in a preferred embodiment, the statistical algorithm includes a logistic regression equation. The accuracy of statistical methods used in accordance with the present invention can be best described by their receiver operating characteristics (ROC). The ROC curve addresses both the sensitivity, the number of true positives, and the specificity, the number of true negatives, of the test. Therefore, sensitivity and specificity values for a given combination of biomarkers are an indication of the accuracy of the assay. For example, if a biomarker combination has sensitivity and specificity values of 80%, out of 100 patients which have stroke, 80 will be correctly identified from the determination of the presence of the particular combination of biomarkers as positive for stroke, while out of 100 patients who have not suffered a stroke 80 will accurately test negative for the disease. If two or more biomarkers are to be used in the diagnostic method a suitable mathematical model, such as logistic regression equation, can be derived. The logistic regression equation might include other variables such as age and gender of patient. The ROC curve can be used to assess the accuracy of the logistic regression model. The logistic regression equation can be used independently or in an algorithm to aid clinical decision making. Although a logistic regression equation is a common mathematical/statistical procedure used in such cases and is preferred in the context of the present invention, other mathematical/statistical procedures can also be used. By way of example, a logistic regression equation applicable

In a clinical setting, an initial step of diagnosing the patient as suffering from, or having suffered from, a haemorrhagic stroke may be carried out using any suitable diagnostic method or technique known in the art, including scanning techniques such as CT and MRI, or assaying a patient's sample for biomarkers of stroke. Haemorrhagic stroke may be diagnosed using a CT scan in combination with GFAP levels. Once haemorrhagic stroke has been ruled out, determining the concentration of at least D-dimer and PARK7 in an *ex vivo* sample obtained from the patient and establishing the significance of the concentration of the biomarkers by comparing the concentration value for each biomarker with a corresponding control value could be used to aid the diagnosis.

In the preferred methods of diagnosing stroke described above control values can be derived from the concentration of corresponding biomarkers in a biological sample obtained from an individual or individuals who have not undergone a stroke. Such control groups or reference groups consist of individuals who have not undergone stroke and may be, for example, healthy individuals, individuals suffering from diseases other than stroke, e.g. stroke mimics. The control group which is chosen may depend on the individual biomarker to be measured. For example, for non-brain-specific biomarkers it may be useful to use a stroke mimic control group, whereas for brain-specific biomarkers it may be useful to use a healthy control group or stroke mimic group. In the case where ischaemic stroke is to be diagnosed or differentiated, the control group may be stroke mimics on their own or in combination with TIA patients. In other cases healthy controls can be grouped with stroke mimics to form a reference group. Alternatively, the control values may correspond to the concentration of each of the biomarker in a sample obtained from the patient prior to the stroke event. In a preferred embodiment, each of the patient and/or control biomarker concentration values is inputted into one or more statistical algorithms to produce an output value that indicates whether a stroke has occurred. The cut-off concentrations or values are derived using a statistical technique; various different methods are available for developing statistical algorithms and are well-known to those skilled in the art. A standard method of biomarker statistical analysis is to use univariate methods to compare biomarker levels in various groups and highlight those biomarkers whose concentrations significantly differ across and between particular groups.

Biomarker concentrations or 'levels' can be determined by contacting the sample with a substrate having probes specific for each of the biomarkers included in the combination of biomarkers. Interactions between a biomarker and its respective probe can be monitored and quantified using various techniques that are well known in the art. Biomarker concentrations are preferably measured in ng/ml. Accordingly, a further aspect of the present disclosure provides a substrate comprising probes specific for D-dimer and probes specific for PARK7, optionally the substrate could further comprise probes specific to any of FABP3, GFAP, GSTP, IL-6, NDKA and sTNFr1. Said substrate may be used according to any of the methods of the current invention for use in aiding the diagnosis of stroke.

The 'level' of a biomarker refers to the amount, expression level or concentration of the biomarker within the sample. This level can be a relative level in comparison to another biomarker or a previous sample. Biomarker levels may be expressed as ratios.

As used herein, the term 'specific', in reference to a probe, means that the probe binds only to one biomarker, with negligible binding to other biomarkers or to other analytes in the biological sample being analysed. This ensures that the integrity of the diagnostic assay and its result is not compromised by additional binding events. Probes can be immobilised on the surface of the substrate, for example covalently immobilised. The substrate can be any substance able to support one or more probes, for example a solid-state device, such as a biochip. A biochip is a planar substrate that may be, for example, mineral or polymer based, but is preferably ceramic. The probe could be a polyclonal or monoclonal antibody, other probes such as aptamers, molecular imprinted polymers, phages, short chain antibody fragments and other antibody-based probes may also be used.

### Methods

### Patient group

The study consisted of 113 patients displaying stroke-like symptoms admitted to the emergency department of KAT general hospital, Athens, Greece. Only patients presenting with stroke for the first time were included. Patients with malignancies, recent history of traumatic brain injury, chronic or end stage liver and renal disease were excluded. Blood samples (serum and EDTA plasma) were taken at the time of admission and at 24, 48, 72, 96 hours and 7 days post admission. Serum and plasma were aliquoted and stored at -80 °C until tested. All patients underwent CT scan upon admission and at 72hrs after the stroke onset. Only patients admitted to the emergency department within 6 hours from the onset of neurological symptoms were included in the study. The mean time from the onset of stroke-symptoms to hospital admission was 3.2 hours. In each patient the presence of arterial hypertension, diabetes mellitus, smoking history, dyslipidemia and ischaemic heart disease were recorded. Stroke severity was measured at the time of admission with the National Institute of Health Stroke Scale (NIHSS). Functional outcome was measured with the modified Rankin scale (mRS) on day 7 and acute stroke patients were categorised into three severity groups (mild, moderate and severe) according to their mRS-score: mild (mRS-score:0-2), moderate (mRS-score:3-4) and severe (mRS-score:5-6). Clinical evaluation (using criteria highlighted in the background section above) and neuroimaging techniques identified 10 haemorrhagic stroke (HS), 53 ischaemic stroke (IS), 13 transient ischaemic attack (TIA) and 37 stroke mimics (M); 79 healthy subjects served as controls (C).

### Sample analysis

The following proteins were tested against EDTA plasma samples of blood obtained on admission from the patients of the study group: PARK7, D-dimer, FABP3, GFAP, GSTP, IL-6, NDKA and STNFr1. The proteins were detected and quantified using multiplexed biochips incorporating biomarker-specific antibodies and the Evidence Investigator (Randox Laboratories Ltd, Crumlin, UK). The simultaneous chemiluminescent sandwich immunoassays were performed according to manufacturer's instructions. A nine-point calibration curve and three reference controls were assayed for each biomarker to allow validation of results.

### Statistical analysis

Single biomarkers were subject to ROC curve analysis to assess sensitivity and specificity. AUC values for biomarker combinations were derived using a Naïve Bayes classifier.

### Results

The data shown in tables 2 and 3 show comparative examples of biomarker combinations to diagnose stroke and differentiate between haemorrhagic stroke (HS), ischaemic stroke (IS), transient ischaemic attack (TIA), stroke mimics (M) and healthy controls (C);

**Table 2**

| **Comparison** | **Biomarker combinations** | **Algorithm AUC output** |
|---|---|---|
| C vs S | D-dimer & IL-6 | 0.962 |
| | D-dimer & PARK7 | 0.955 |
| | D-dimer, FABP3 & PARK7 | 0.966 |
| | D-dimer, IL-6 & PARK7 | 0.965 |
| | D-dimer, FABP3, IL-6, PARK7 & sTNFr1 | 0.973 |
| M vs S | D-dimer & PARK7 | 0.883 |
| | FABP3 & PARK7 | 0.871 |
| | D-dimer, FABP3 & PARK7 | 0.894 |
| | D-dimer, IL-6 & PARK7 | 0.883 |
| | D-dimer, FABP3, GFAP &PARK7 | 0.897 |
| C & M vs S | D-dimer & FABP3 | 0.920 |
| | D-dimer & PARK7 | 0.927 |
| | D-dimer, IL-6 & PARK7 | 0.934 |
| | D-dimer, PARK7 & sTNFr1 | 0.933 |
| | D-dimer, FABP3, GFAP, IL-6, PARK7 & sTNFr1 | 0.942 |
| C vs IS | D-dimer & IL-6 | 0.960 |
| | D-dimer & FABP3 | 0.959 |
| | D-dimer, FABP3 & IL-6 | 0.968 |
| | D-dimer, FABP3 & PARK7 | 0.959 |
| | D-dimer, FABP3, IL-6 & sTNFr1 | 0.973 |
| M vs IS | D-dimer & PARK7 | 0.886 |
| | FABP3 & PARK7 | 0.880 |
| | D-dimer, FABP3 & PARK7 | 0.893 |
| | D-dimer, IL-6 & PARK7 | 0.884 |
| C & M vs IS | D-dimer & FABP3 | 0.927 |
| | D-dimer & PARK7 | 0.929 |
| | D-dimer, FABP3 & IL-6 | 0.935 |
| | D-dimer, FABP3 & PARK7 | 0.933 |
| | D-dimer, FABP3, IL-6, PARK7 & sTNFr1 | 0.941 |
| M & TIA vs IS | D-dimer & PARK7 | 0.798 |
| | D-dimer, FABP3 & PARK7 | 0.810 |
| | D-dimer, FABP3, PARK7 & sTNFr1 | 0.804 |
| C & M & TIA vs IS | D-dimer & IL-6 | 0.885 |
| | D-dimer & PARK7 | 0.886 |
| | D-dimer, IL-6 & PARK7 | 0.894 |
| | D-dimer, FABP3 & PARK7 | 0.884 |

**Table 3**

| **Comparison** | **Biomarker combinations** | **Algorithm AUC output** |
|---|---|---|
| IS vs HS | GFAP, IL-6, PARK7 & sTNFr1 | 0.918 |
| TIA vs HS | GFAP & IL-6 | 0.859 |
| IS & TIA vs HS | FABP3, GFAP, IL-6, PARK7 & sTNFr1 | 0.890 |

**Table 4 Statistical analysis of healthy control cohort (HC) vs stroke mimic cohort (mimics) and ischaemic stroke cohort vs HC, mimics and TIA + mimics.**

| Biomarker | Bivariate analysis HC vs mimics | ROC IS vs HC | | | ROC IS vs mimics | | | ROC IS vs TIA + mimics | | |
|---|---|---|---|---|---|---|---|---|---|---|
| | | Sens | Spec | AUC | Sens | Spec | AUC | Sens | Spec | AUC |
| h-FABP | P = 0.0032 | 85% | 95% | 0.9315 | 58% | 89% | 0.8389 | 56% | 80% | 0.7665 |
| D-dimer | P < 0.0001 | 85% | 95% | 0.9667 | 69% | 89% | 0.8559 | 61% | 80% | 0.7922 |
| GFAP | P = 0.5331 | NA | | | NA | | | NA | | |
| GSTPi | NA | NA | | | NA | | | NA | | |
| IL-6 | P = 0.0008 | 61% | 95% | 0.9126 | 56% | 76% | 0.7457 | 56% | 74% | 0.7074 |
| NDKA | P = 0.0839 | NA | | | NA | | | NA | | |
| PARK-7 | P = 0.4143 | NA | | | NA | | | NA | | |
| sTNFR1 | P = 0.0058 | 70% | 95% | 0.8881 | 63% | 89% | 0.8146 | 61% | 80% | 0.7587 |

**Table 5 Biomarker median values (units in first column) of HC, mimic and mimic + TIA cohorts and % increase (inc.) of HC vs mimic and HC vs mimic + TIA. Column 4 displays cut-off values used to derive the sensitivities and specificities highlighted in Table 4.**

| Biomarker | Median | | | Median | | | Cut-off | |
|---|---|---|---|---|---|---|---|---|
| | HC | mimic | inc. | HC | mim + TIA | inc. | IS vs mim | IS vs mim+TIA |
| h-FABP (ng/ml) | 1.57 | 2.24 | 43% | 1.57 | 2.70 | 72% | 4.78 | 5.00 |
| D-dimer (ng/ml) | 46 | 77 | 67% | 46 | 102 | 122% | 201 | 217.6 |
| IL-6 (pg/ml) | 1.00 | 1.71 | 71% | 1.00 | 3.02 | 202% | 5.98 | 6.07 |
| sTNFR1 (ng/ml) | 1.48 | 1.59 | 7% | 1.48 | 1.66 | 12% | 2.37 | 2.55 |

## Claims

1. A method of aiding the diagnosis of stroke in a patient suspected of having had or currently suffering a stroke, said method comprising;
i) Determining the concentration of Parkinson disease protein 7 (PARK7) and D-dimer in an ex vivo blood, serum or plasma sample obtained from the patient;
ii) Establishing the statistical significance of the concentration of the biomarkers by comparison to a control value, wherein each of the biomarker concentration values are inputted into a statistical algorithm or algorithms to produce an output value that correlates with diagnosis of stroke.

2. The method of claim 1 wherein the concentration of one or more additional biomarker selected from the list consisting of Fatty acid-binding protein 3 (FABP3), Glial fibrillary acid protein (GFAP), Glutathione S-transferase P (GSTP), interleukin 6 (IL-6), Nucleoside diphosphate kinase A (NDKA) and soluble tumour necrosis factor receptor 1 (sTNFr1) is also determined.

3. The method according to claim 2, wherein any of the following concentrations are determined
i) PARK7, D-dimer and FABP3
ii) PARK7, D-dimer and IL-6
iii) PARK7, D-dimer and sTNFr1
iv) PARK7, D-dimer and GFAP
v) PARK7, D-dimer, FABP3 and sTNFr1
vi) PARK7, D-dimer, FABP3 and IL-6
vii) PARK7, D-dimer, FABP3, sTNFr1 and IL-6
viii) D-dimer, FABP3, GFAP and PARK7
ix) D-dimer, FABP3, GFAP, IL-6, PARK7 and sTNFr1

4. The method according to claims 1-3 for the diagnosis of ischaemic stroke.

## Patentansprüche

1. Verfahren zur Unterstützung der Schlaganfall diagnose bei einem Patienten, bei dem der Verdacht besteht, einen Schlaganfall gehabt zu haben oder gegenwärtig erleidet, wobei das Verfahren umfasst:
i) Bestimmen der Konzentration von Parkinson-Krankheits protein 7 (PARK7) und D-Dimer in einer ex vivo Blut-, Serum- oder Plasmaprobe, die dem Patienten entnommen wurde;
ii) Ermitteln der statistischen Signifikanz der Konzentration der Biomarker durch Vergleich mit einem Kontrollwert, wobei jeder der Biomarker-Konzentrationswerte in einen statistischen Algorithmus oder Algorithmen eingegeben wird, um einen Ausgabewert zu erzeugen, der mit der Diagnose eines Schlaganfalls korreliert.

2. Verfahren nach Anspruch 1, wobei die Konzentration eines oder mehrerer zusätzlicher Biomarker ausgewählt aus der Liste bestehend aus Fettsäure-bindendes Protein 3 (FABP3), Gliafibrilläres Säureprotein (GFAP), Glutathion-S-Transferase P (GSTP), Interleukin 6 (IL-6), Nukleosiddiphosphatkinase A (NDKA) und löslicher Tumornekrosefaktor-Rezeptor 1 (sTNFr1) werden ebenfalls bestimmt.

3. Verfahren nach Anspruch 2, wobei eine der folgenden Konzentrationen bestimmt werden -
i) PARK7, D-Dimer und FABP3
ii) PARK7, D-Dimer und IL-6
iii) PARK7, D-Dimer und sTNFr1
iv) PARK7, D-Dimer und GFAP
v) PARK7, D-Dimer, FABP3 und sTNFr1
vi) PARK7, D-Dimer, FABP3 und IL-6
vii) PARK7, D-Dimer, FABP3, sTNFr1 und IL-6
viii) D-Dimer, FABP3, GFAP und PARK7
ix) D-Dimer, FABP3, GFAP, IL-6, PARK7 und sTNFr1

4. Verfahren nach den Ansprüchen 1-3 zur Diagnose des ischämischen Schlaganfalls.

## Revendications

1. Méthode d'aide au diagnostic d'un AVC chez un patient suspecté d'avoir subi ou souffrant actuellement d'un AVC, ladite méthode comprenant:
i) Détermination de la concentration de la protéine de la maladie de Parkinson 7 (PARK7) et des D-dimères dans un échantillon de sang, de sérum ou de plasma ex vivo obtenu à partir du patient;
ii) établir la signification statistique de la concentration des biomarqueurs par comparaison à une valeur de contrôle, chacune des valeurs de concentration de biomarqueurs étant entrée dans un ou des algorithmes statistiques pour produire une valeur de sortie qui correspond au diagnostic d'AVC.

2. Procédé selon la revendication 1 dans lequel la concentration d'un ou plusieurs biomarqueurs supplémentaires choisis dans la liste constituée par Fatty acid-binding protein 3 (FABP3), Glial fibrillary acid protein (GFAP), Glutathion S-transferase P (GSTP), interleukine 6 (IL-6), la nucléoside diphosphate kinase A (NDKA) et le récepteur du facteur de nécrose tumorale soluble 1 (sTNFr1) sont également déterminés.

3. Procédé selon la revendication 2, dans lequel l'une quelconque des concentrations suivantes est déterminée -
i) PARK7, D-dimères et FABP3
ii) PARK7, D-dimères et IL-6
iii) PARK7, D-dimères et sTNFr1
iv) PARK7, D-dimères et GFAP
v) PARK7, D-dimères, FABP3 et sTNFr1
vi) PARK7, D-dimères, FABP3 et IL-6
vii) PARK7, D-dimères, FABP3, sTNFr1 et IL-6
viii) D-dimères, FABP3, GFAP et PARK7
ix) D-dimères, FABP3, GFAP, IL-6, PARK7 et sTNFr1

4. Méthode selon les revendications 1 à 3 pour le diagnostic d'AVC ischémique.
